(19) Europäisches Patentamt
European Patent Office
Office européen des brevets

(11) Numéro de publication: **0 490 750 B1**

(12) **FASCICULE DE BREVET EUROPEEN**

(45) Date de publication de fascicule du brevet: **21.06.95**

(51) Int. Cl.[6]: **A61K 7/48**, A61K 7/00

(21) Numéro de dépôt: **91403330.3**

(22) Date de dépôt: **09.12.91**

Le dossier contient des informations techniques présentées postérieurement au dépôt de la demande et ne figurant pas dans le présent fascicule.

# (54) Composition cosmétique liquide à deux phases, contenant au moins un dialkylphtalate, des particules insolubles et un agent équilibrant les densités.

(30) Priorité: **12.12.90 FR 9015580**

(43) Date de publication de la demande: **17.06.92 Bulletin 92/25**

(45) Mention de la délivrance du brevet: **21.06.95 Bulletin 95/25**

(84) Etats contractants désignés: **AT BE CH DE DK ES FR GB GR IT LI NL SE**

(56) Documents cités:
**WO-A-82/04189**
**DE-A- 3 009 763**
**FR-A- 893 112**
**US-A- 3 920 883**
**US-A- 4 767 741**

(73) Titulaire: **L'OREAL**
**14, rue Royale**
**F-75008 Paris (FR)**

(72) Inventeur: **Marion, Hélène**
**75 rue de Patay**
**F-75013 Paris (FR)**
Inventeur: **Louvet, Nathalie**
**13 rue Du Guesclin**
**F-94240 L'Hay-les-Roses (FR)**
Inventeur: **Lukassen, Liliane**
**Résidence de l'Hermitage,**
**24 Allée d'Alsace**
**F-94550 Chevilly-Larue (FR)**

(74) Mandataire: **Casalonga, Axel et al**
**BUREAU D.A. CASALONGA - JOSSE**
**Morassistrasse 8**
**D-80469 München (DE)**

EP 0 490 750 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention a pour objet une composition liquide à deux phases, comprenant (a) une phase aqueuse, (b) une phase huileuse à base de dialkylphtalate, (c) des particules solides insolubles dans la phase aqueuse et dans la phase huileuse et (d) un agent équilibrant les densités.

Le document FR 893 112 décrit certains dialkylphtalates, leur procédé de préparation et leur utilisation dans le domaine de la cosmétique et de la pharmacie, dans des crèmes grasses et des suppositoires notamment.

On connaît des compositions cosmétiques liquides à deux phases, contenant une phase huileuse et une phase aqueuse, ces deux phases se trouvant l'une au-dessus de l'autre. Au moment de l'emploi, on obtient une dispersion par agitation. Une telle composition à deux phases stratifiées se prête assez mal à la formation d'une suspension homogène par agitation ou secouage et la partie huileuse supérieure a tendance à être consommée en quantité plus grande que la partie aqueuse.

La demande de brevet français n° 2.208.642 (SHISEIDO) enseigne une composition liquide, comprenant (1) une phase huileuse, (2) une phase aqueuse, (3) un liquide organique miscible à la phase aqueuse, mais non miscible à la phase huileuse, (4) des particules solides finement divisées insolubles dans les liquides (1), (2) et (3), ces particules étant adsorbées à l'interface de (1) et (2). Les compositions décrites comportent un alcool.

Le brevet US 4.767.741, de KOMOR (AVON PRODUCTS) enseigne une composition cosmétique liquide à deux phases, similaire à celle de SHISEIDO avec la différence que les particules solides non miscibles sont formées par précipitation in situ.

Ces deux brevets utilisent comme liquide organique (3) un alcool de faible poids moléculaire et en particulier l'éthanol.

Cependant, la présence d'un alcool de faible poids moléculaire tel que l'éthanol, est souvent irritante et un besoin existe pour une composition cosmétique aqueuse exempte d'alcool.

Par ailleurs, les deux brevets susmentionnés utilisent comme phase huileuse la paraffine liquide, du squalane, des acides gras supérieurs, des esters de mono-acides ou de diacides gras, des huiles végétales ou animales, des alcools gras, des polyalkylèneglycols ou des huiles synthétiques. Aucun de ces deux brevets n'enseigne l'utilisation des dialkylphtalates.

On a découvert que la présence de dialkylphtalate dans une composition cosmétique liquide aqueuse communique à la composition des qualités cosmétiques appréciables, notamment des propriétés filmogènes et de douceur. En effet, le dialkylphtalate communique à la peau un toucher doux.

On a également constaté que la présence du dialkylphtalate favorise la permanence du parfum dans la composition ainsi que sur la peau même en l'absence d'alcool.

Enfin, l'utilisation d'un dialkylphtalate permet d'obtenir une composition liquide à deux phases présentant un aspect esthétiquement attrayant.

L'invention a pour objet une composition cosmétique liquide exempte d'alcool du type à deux phases, comprenant (a) une phase aqueuse, (b) une phase huileuse comprenant un ou plusieurs dialkylphtalate(s), (c) des particules solides insolubles dans la phase aqueuse et dans la phase huileuse et (d) un ou plusieurs agent(s) équilibrant les densités; la phase huileuse se présentant sous la forme de billes qui, par agitation, se dispersent de façon homogène dans la phase aqueuse et au repos se reconstituent au fond du récipient sous forme de perles. Cette composition à deux phases, exempte d'alcool, présente un aspect esthétiquement attrayant.

D'autres objets de l'invention apparaîtront à la lecture de la description et des exemples.

Les dialkylphtalates utilisés dans les compositions ont pour formule générale :

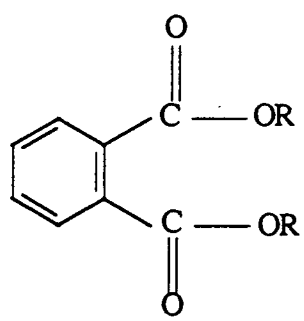

où R est un reste alkyle en $C_1$-$C_4$, en particulier un reste méthyle, éthyle, butyle.

La phase aqueuse peut être constituée par de l'eau déminéralisée stérile ou par une eau florale telle que de l'eau de rose, de l'eau de bleuet, de l'eau de camomille ou de l'eau de tilleul.

Les particules solides insolubles dans la phase huileuse ainsi que dans la phase aqueuse, ont une dimension inférieure à 10 µm. On peut utiliser toute particule solide insoluble dans les deux phases et qui se maintient à l'interface huile/eau.

Les particules solides insolubles sont choisies de préférence dans le groupe formé par les matières minérales et organiques suivantes : oxyde de fer, dioxyde de titane, oxyde d'antimoine, oxyde de magnésium, alumine, oxyde de zinc, peroxyde de zinc, aluminate de calcium, acide silicique, silico-aluminate de magnésium, talc, mica, kaolin colloïdal, bentonite, laurate de zinc, chlorure de polyvinyle, nacre, noir de carbone, lanoline.

La composition cosmétique à deux phases selon l'invention comprend également un ou plusieurs agents équilibrant les densités qui ont pour but d'équilibrer les densités de la phase de dialkylphtalate et celle de la phase aqueuse. Ces agents équilibrant les densités sont choisis dans le groupe formé par les produits solubles dans la phase aqueuse, les produits solubles dans le dialkylphtalate et leurs mélanges. La fonction de l'agent équilibrant les densités est d'augmenter la densité de la phase aqueuse en augmentant sa masse sans pratiquement changer son volume ou bien de diminuer la densité du dialkylphtalate en augmentant son volume sans changer considérablement sa masse.

Comme exemple d'agent équilibrant les densités, soluble dans les dialkylphtalates, on peut citer les huiles, notamment les adipates tels que le dioctyladipate, les myristates tels que l'isopropylmyristate, les palminates tels que l'octylpalmitate, les stéarates tels que l'isopropylstéarate, des vitamines telles que la vitamine A, la vitamine E, la vitamine F, des huiles telles que l'huile de tournesol, l'huile de poisson, le tétra éthyl-2 hexanoate de pentaérythritol et produits similaires.

Comme agent équilibrant les densités, soluble dans l'eau, on peut citer les sels minéraux ou organiques hydrosolubles tels que le phosphate trisodique, le phosphate disodique, le phosphate monosodique, le métabisulfite de sodium, le sulfate de magnésium, le sulfate de sodium, le phosphate monopotassique, le phosphate dipotassique, le phosphate tripotassique, le chlorure de sodium, le chlorure de potassium, les citrates mono-, di- et trisodiques, et en général les sels minéraux solubles dans l'eau.

Comme agent équilibrant les densités, soluble dans l'eau, on peut également utiliser un composant cosmétique, par exemple un conservateur, un filtre UV, un agent tampon, un agent d'éclat du teint et en général tout composé soluble dans l'eau qui augmente la densité de l'eau.

On peut utiliser à la fois un agent équilibrant les densités, soluble dans l'eau et un agent équilibrant les densités, soluble dans le dialkylphtalate.

Dans la composition liquide à deux phases, selon l'invention, la phase à base de dialkylphtalate se présente sous forme de billes qui, par agitation, se dispersent sous forme de micro-billes dans la phase aqueuse et se reconstituent au repos au fond de la phase aqueuse. Les particules solides insolubles se placent à l'interface des billes d'huile contenant le dialkylphtalate et de l'eau.

Les particules solides insolubles et les agents équilibrant les densités favorisent la formation et la stabilité des billes d'huile dans l'eau au repos.

La composition cosmétique selon l'invention renferme de 0,5 à 15 % et de préférence de 2 à 10% en poids de dialkylphtalate, de 1 à 12% en poids d'agent équilibrant les densités et de 0,005 à 0,5% et de préférence de 0,01 à 0,05% en poids de particules solides insolubles dans les deux phases, du poids total de la composition. La phase aqueuse représente de 78 à 99,5% et de préférence de 90 à 95% en poids du poids total de la composition.

La composition cosmétique liquide à deux phases peut également renfermer les adjuvants habituellement utilisés dans la cosmétique. Selon leur nature hydrophile ou lipophile, ces adjuvants seront dissous dans la phase aqueuse ou dans la phase de dialkylphtalate. Parmi ces adjuvants, on peut citer les parfums, les agents conservateurs, les séquestrants, les colorants; les agents adoucissants tels que l'allantoïne, les extraits de plante; les extraits de fruits; les agents hydratants tels que la glycérine, l'hydroxyproline, les agents tampon, les humectants tels que le butylèneglycol, les filtres UV liposolubles, les huiles, les vitamines, notamment la vitamine B5, la vitamine E, les agents d'éclat du teint tels que la guanosine, etc.

Pour préparer la composition à deux phases, on prépare d'abord la phase aqueuse en dissolvant dans l'eau l'agent équilibrant les densités et les adjuvants solubles dans l'eau. On disperse dans cette phase aqueuse, soit à froid, soit en chauffant légèrement, les particules solides insolubles dans l'eau et on y verse ensuite la phase contenant le dialkylphtalate, les agents équilibrant la densité, solubles dans le dialkylphtalate et les autres adjuvants liposolubles (huiles, parfums, etc.). On agite environ une heure à une heure et demi et en laissant reposer, on obtient deux phases.

L'invention sera mieux comprise à l'aide des exemples non limitatifs ci-après.

EXEMPLE 1

**BAUME APRES-RASAGE**

| | | % en poids |
|---|---|---|
| - Diéthylphtalate | | 4 |
| - Nacre | | 0,02 |
| - Glycérine | | 3 |
| - Allantoïne | | 0,2 |
| - Chlorure de sodium | | 3 |
| - Butylène glycol | | 1 |
| - Citrate trisodique | | 2 |
| - Conservateur | | 0,2 |
| - Tétra-éthyl 2-hexanoate de pentaerytritol | | 1 |
| - Parfum | | 0,05 |
| - Eau | qsp | 100 |

EXEMPLE 2

**COMPOSITION TONIOUE**

| | | % en poids |
|---|---|---|
| - Diéthylphtalate | | 6 |
| - Mica | | 0,02 |
| - Citrate trisodique | | 3 |
| - Chlorure de sodium | | 3 |
| - Sulfate de magnésium | | 2 |
| - Allantoïne | | 0,05 |
| - Conservateurs | | 0,3 |
| - Parfum | | 1 |
| - Eau | qsp | 100 |

EXEMPLE 3

**BAUME APRES-RASAGE**

| | | % en poids |
|---|---|---|
| - Diéthylphtalate | | 2 |
| - Diisotridécyle myristate | | 0,5 |
| - Nacre | | 0,04 |
| - Citrate de sodium | | 2 |
| - Chlorure de sodium | | 1 |
| - Sel disodique de l'acide éthylène diamine tetra-acétique (EDTA) | | 2 |
| - Acide 2-hydroxy 4-méthoxybenzophénone 5-sulfurique (Filtre UV) | | 0,05 |
| - Conservateurs | | 0,3 |
| - Allantoïne | | 0,9 |
| - Eau | qsp | 100 |

EXEMPLE 4

**COMPOSITION TONIOUE**

| | | % en poids |
|---|---|---|
| - Diéthylphtalate | | 12 |
| - Dioctyladipate | | 9 |
| - Nacre | | 0,02 |
| - Parfum | | 0,1 |
| - Glycérine | | 1 |
| - Chlorure de sodium | | 2 |
| - Conservateurs | | 0,3 |
| - Eau | qsp | 100 |

EXEMPLE 5

**COMPOSITION TONIOUE**

| | | % en poids |
|---|---|---|
| - Dibutylphtalate | | 4 |
| - Isotridécylmyristate | | 5 |
| - Nacre | | 0,02 |
| - Parfum | | 0,1 |
| - Glycérine | | 5 |
| - Chlorure de sodium | | 1 |
| - Phosphate monopotassique | | 0,5 |
| - Phosphate dipotassique | | 0,5 |
| - Conservateurs | | 0,3 |
| - Eau | qsp | 100 |

**COMPARAISON DES PROPRIETES COSMETIOUES DES COMPOSITIONS AVEC ET SANS DIETHYLPHTALATE**

On a préparé les trois compositions cosmétiques liquides diphasées ci-après :

| Composition 1 | | % en poids |
|---|---|---|
| - Diéthylphtalate | | 5 |
| - Mica | | 0,02 |
| Chlorure de sodium | | 9 |
| - Parfum | | 0,05 |
| - Eau | qsp | 100 |

| Composition 2 | | |
|---|---|---|
| - Tétra éthyl 2-hexanoate de pentaérythritol | | 5 |
| - Mica | | 0,02 |
| - Chlorure de sodium | | 9 |
| - Parfum | | 0,05 |
| - Eau | qsp | 100 |

| Composition 3 | | |
|---|---|---|
| - Alcool éthylique | | 5 |
| - Mica | | 0,02 |
| - Chlorure de sodium | | 9 |
| - Parfum | | 0,05 |
| - Eau | qsp | 100 |

La composition 1, selon l'invention, contient du diéthylphtalate, la composition 2 contient à la place du diéthylphtalate du tétraéthyl 2-hexanoate de pentaérythritol qui est un adoucissant utilisé en cosmétique, et la composition 3 contient à la place du diéthylphtalate de l'alcool éthylique.

On a soumis cette composition à un panel de 12 femmes qui ont eu à juger en aveugle les trois compositions suivantes, en ce qui concerne l'effet filmogène, l'effet moulant (pénétration rapide), la sensation (désagréable) de gras, l'effet non collant, la fraîcheur, la sensation de douceur après application, l'appréciation du parfum, la tenue du parfum.

Les résultats figurent sur le tableau ci-après.

| | Composition 1 | Composition 2 | Composition 3 |
|---|---|---|---|
| Effet filmogène | 12 + | 12 + | 12 - |
| Effet mouillant (pénétration rapide) | 12 + | 12 - | 12 + |
| Sensation (désagréable) de gras | 4 + ; 8 - | 12 + | 2 + ; 10 - |
| Effet non collant | 10 + ; 2 - | 6 + ; 6 - | 10 + ; 2 - |
| Fraîcheur | 12 + | 12 - | 12 + |
| Sensation de douceur après application | 10 + ; 2 - | 8 + ; 4 - | 6 + ; 6 - |
| Perception du parfum | 12 + | 12 - | 10 + ; 2 - |
| Tenue du parfum | 12 + | 12 - | 12 - |

Sur ce tableau, le signe + indique une appréciation favorable et le signe - une appréciation défavorable. Les chiffres indiquent le nombre de femmes qui partagent l'appréciation favorable, respectivement défavorable.

On constate que la composition 1, selon l'invention, présente un effet filmogène, une pénétration rapide, une sensation de fraîcheur, une bonne perception du parfum et une bonne tenue du parfum, pour les 12 femmes; que 8 femmes sur 12 ressentent l'absence d'effet gras; que 10 femmes sur 12 trouvent la composition non collante et 10 femmes sur 12 trouvent que la peau est douce après l'application.

Pour la composition 2, 12 femmes notent un effet gras désagréable, l'absence de fraîcheur, un manque de pénétration rapide, une faible perception et mauvaise tenue du parfum, 6 femmes sur 12 trouvent un effet collant, 8 femmes sur 12 trouvent que la peau est douce après application.

Pour la composition 3, 12 femmes sur 12 trouvent une bonne pénétration, une sensation de fraîcheur, mais une absence d'effet filmogène et une mauvaise tenue du parfum, et seulement 6 femmes sur 12 trouvent que la peau est douce après application.

On constate par conséquent que la composition 1, selon l'invention, contenant du dialkylphtalate allie les qualités d'effet filmogène et de douceur après application aux qualités de fraîcheur, de pénétration rapide, de perception et bonne tenue du parfum et que la grande majorité des femmes trouvent la composition non collante et peu grasse.

La composition selon l'invention a par conséquent été jugée nettement supérieure par rapport aux compositions témoins 2 et 3.

## Revendications

1. Composition cosmétique liquide du type à deux phases, comprenant (a) une phase aqueuse, (b) une phase huileuse, (c) des particules solides insolubles dans la phase aqueuse et dans la phase huileuse, la phase huileuse se présentant sous la forme de billes,
caractérisée en ce que la composition est exempte d'alcool, et
(i) la phase huileuse sous forme de billes comprend un ou plusieurs dialkylphtalates de formule :

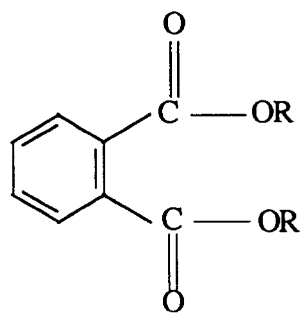

où R est un reste alkyle en $C_1$-$C_4$;
(ii) la composition comprend également un ou plusieurs agents équilibrant les densités.

2. Composition selon la revendication 1, caractérisée en ce que les particules solides insolubles sont choisies dans le groupe formé par les matières minérales et organiques suivantes : oxyde de fer, dioxyde de titane, oxyde d'antimoine, oxyde de magnésium, alumine, oxyde de zinc, peroxyde de zinc, aluminate de calcium, acide silicique, silicoaluminate de magnésium, talc, mica, kaolin colloïdal, bentonite, laurate de zinc, chlorure de polyvinyle, nacre, noir de carbone, lanoline.

3. Composition selon la revendication 1 ou 2, caractérisée en ce que le ou les agents équilibrant les densités sont choisis dans le groupe formé par les produits solubles dans la phase aqueuse, les produits solubles dans la phase huileuse et leurs mélanges.

4. Composition selon l'une quelconque des revendications 1 à 3, caractérisée en ce qu'elle contient de 0,5 à 15% et de préférence de 2 à 10% en poids de dialkylphtalate.

5. Composition selon l'une quelconque des revendications 1 à 4, caractérisée en ce qu'elle contient de 0,005 à 0,5% et de préférence de 0,01 à 0,05% en poids de particules solides insolubles.

6. Composition selon l'une quelconque des revendications 2 à 5, caractérisée en ce qu'elle contient de 1 à 12% en poids d'agent équilibrant les densités.

7. Composition selon la revendication 3, caractérisée en ce que l'agent équilibrant les densités soluble dans la phase huileuse, est choisi parmi les huiles telles que les myristates, les adipates, les palmitates, les stéarates, les vitamines A, E et F, l'huile de toumesol, l'huile de poisson et le tétraéthyl-2 hexanoate de pentaérythritol.

8. Composition selon l'une quelconque des revendications 2 à 5, caractérisée en ce que l'agent équilibrant les densités, soluble dans l'eau, est choisi plus particulièrement dans le groupe formé par les sels minéraux et organiques, les conservateurs, les filtres UV et les agents tampon, les agents d'éclat du teint.

9. Composition selon l'une quelconque des revendications 1 à 7, caractérisée en ce qu'elle renferme également au moins un adjuvant cosmétique choisi dans le groupe formé par les parfums, les agents conservateurs, les colorants, les agents adoucissants, les agents hydratants, les agents tampons, les humectants, les filtres UV liposolubles, les huiles, les vitamines, les produits séquestrants et les agents d'éclat du teint.

## Claims

1. Two-phase type liquid cosmetic composition comprising (a) an aqueous phase, (b) an oily phase, (c) solid particles which are insoluble in the aqueous phase and in the oily phase, the oily phase being in the form of beads, characterised in that the composition is alcohol-free and:
   (i) the oily phase in the form of beads comprises one or more dialkylphthalates of formula:

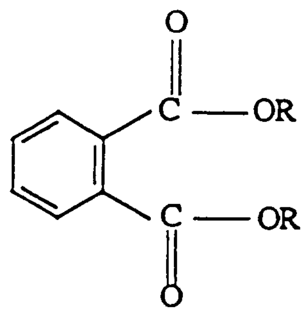

   in which R is a $C_1$-$C_4$ alkyl residue:
   (ii) the composition also comprises one or more agents which balance the densities.

2. Composition according to Claim 1, characterised in that the insoluble solid particles are chosen from the group consisting of the following inorganic and organic materials: iron oxide, titanium dioxide, antimony oxide, magnesium oxide, alumina, zinc oxide, zinc peroxide, calcium aluminate, silicic acid, magnesium silicoaluminate, talc, mica, colloidal kaolin, bentonite, zinc laurate, polyvinyl chloride, nacre, carbon black and lanolin.

3. Composition according to Claim 1 or 2, characterised in that the agents which balance the densities are chosen from the group consisting of the aqueous-phase-soluble products, the oily-phase-soluble products and their mixtures.

4. Composition according to any one of Claims 1 to 3, characterised in that it contains 0.5 to 15 % and, preferably, 2 to 10 % by weight of dialkylphthalate.

5. Composition according to any one of Claims 1 to 4, characterised in that it contains 0.005 to 0.5 % and, preferably, 0.01 to 0.05 % by weight of insoluble solid particles.

6. Composition according to any one of Claims 2 to 5, characterised in that it contains 1 to 12 % by weight of agent which balances the densities.

7. Composition according to Claim 3, characterised in that the oily-phase-soluble agent which balances the densities is chosen from oils such as myristates, adipates, palmitates, stearates, vitamins A, E and F, sunflower oil, fish oil and pentaerythritol 2-tetraethylhexanoate.

8. Composition according to any one of Claims 2 to 5, characterised in that the water-soluble agent which balances the densities is chosen, more particularly, from the group consisting of inorganic and organic salts, preservatives, UV screens, buffers and complexion enhancing agents.

9. Composition according to any one of Claims 1 to 7, characterised in that it also contains at least one cosmetic adjuvant chosen from the group consisting of perfumes, preserving agents, colorants, softening agents, moisturising agents, buffers, humectants, fatsoluble UV screens, oils, vitamins, sequestering products and complexion-enhancing agents.

## Patentansprüche

1. Kosmetische flüssige Zusammensetzung vom Typ aus zwei Phasen, enthaltend (a) eine wässrige Phase, (b) eine ölige Phase, (c) Feststoffteilchen, die in der wässrigen Phase und in der öligen Phase

unlöslich sind, wobei die ölige Phase in Form von Kugeln vorliegt, dadurch **gekennzeichnet**, daß die Zusammensetzung keinen Alkohol aufweist, und daß

(i) die ölige Phase in Form von Kugeln ein oder mehrere Dialkylphthalate der Formel:

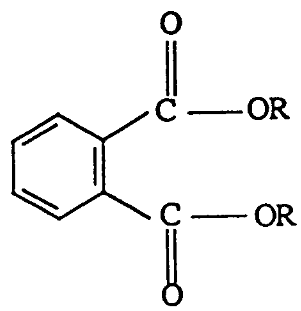

worin R ein $C_{1-4}$-Alkylrest ist, und

(ii) die Zusammensetzung auch ein oder mehrere Dichteausgleichsmittel enthalten.

**2.** Zusammensetzung gemäß Anspruch 1, dadurch **gekennzeichnet**, daß die unlöslichen Feststoffteilchen aus der Gruppe aus den folgenden mineralischen und organischen Stoffen ausgewählt sind: Eisenoxid, Titandioxid, Antimonoxid, Magnesiumoxid, Aluminiumoxid, Zinkoxid, Zinkperoxid, Calciumaluminat, Kieselsäure, Magnesiumsilikoaluminat, Talk, Glimmer, kolloidales Kaolin, Bentonit, Zinklaurat, Polyvinylchlorid, Perlmutt, Kohlenstoffschwarz, Lanolin.

**3.** Zusammensetzung gemäß Anspruch 1 oder 2, dadurch **gekennzeichnet**, daß die Dichteausgleichsmittel aus der Gruppe aus in der wässrigen Phase löslichen Produkten, in der öligen Phase löslichen Produkten sowie aus deren Mischungen ausgewählt sind.

**4.** Zusammensetzung gemäß jedem der Ansprüche 1 bis 3, dadurch **gekennzeichnet**, daß sie 0,5 bis 15 und vorzugsweise 2 bis 10 Gew.% Dialkylphthalat enthält.

**5.** Zusammensetzung gemäß jedem der Ansprüche 1 bis 4, dadurch **gekennzeichnet**, daß sie 0,005 bis 0,5 und vorzugsweise 0,01 bis 0,05 Gew.% unlösliche Feststoffteilchen enthält.

**6.** Zusammensetzung gemäß jedem der Ansprüche 2 bis 5, dadurch **gekennzeichnet**, daß sie 1 bis 12 Gew.% Dichteausgleichsmittel enthält.

**7.** Zusammensetzung gemäß Anspruch 3, dadurch **gekennzeichnet**, daß das in der öligen Phase lösliche Dichteausgleichsmittel aus Ölen wie Myristaten, Adipaten, Palmitaten und Stearaten, aus den Vitaminen A, E und F, aus Sonnenblumenöl, Fischöl und aus Pentaerythrityltetra-2-ethylhexanoat ausgewählt ist.

**8.** Zusammensetzung gemäß jedem der Ansprüche 2 bis 5, dadurch **gekennzeichnet**, daß das in Wasser lösliche Dichteausgleichsmittel insbesondere aus der Gruppe aus mineralischen und organischen Salzen, Konservierungsmitteln, UV-Filterstoffen, Puffermitteln und aus Glanzmitteln für den Teint ausgewählt ist.

**9.** Zusammensetzung gemäß jedem der Ansprüche 1 bis 7, dadurch **gekennzeichnet**, daß

sie auch mindestens einen kosmetischen Hilfsstoff aufweist, ausgewählt aus der Gruppe aus Parfüm-Produkten, Konservierungsmitteln, Färbemitteln, weichmachenden Mitteln, hydratisierenden Mitteln, Puffermitteln, Feuchtigkeitsmitteln, fettlöslichen UV-Filterstoffen, Ölen, Vitaminen, Sequestrierungsprodukten sowie aus Glanzmitteln für den Teint.